(19) Europäisches Patentamt
European Patent Office
Office européen des brevets

(11) Veröffentlichungsnummer: **0 338 435**
**A1**

(12) **EUROPÄISCHE PATENTANMELDUNG**

(21) Anmeldenummer: **89106625.0**

(22) Anmeldetag: **13.04.89**

(51) Int. Cl.4: **C07D 207/273 , C07D 491/04 , C07D 409/06 , C07D 317/32 , A61K 31/40**

Patentansprüche für folgende Vertragsstaaten: ES + GR.

(30) Priorität: **21.04.88 DE 3813416**

(43) Veröffentlichungstag der Anmeldung: **25.10.89 Patentblatt 89/43**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Kretzschmar, Gerhard, Dr.**
**Falkenstrasse 71**
**D-6232 Bad Soden am Taunus(DE)**
Erfinder: **Hock, Franz, Dr.**
**Altstadt 19**
**D-6110 Dieburg(DE)**

(54) **3.4-Dihydroxypyrrolidin-2-on-Derivate, Verfahren zu ihrer Herstellung, diese enthaltende Mittel und ihre Verwendung sowie die bei der Herstellung anfallenden Zwischenprodukte.**

(57) 3,4-Dihydroxypyrrolidin-2-on-Derivate der Formel I

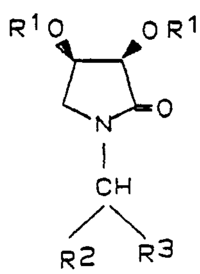

( I )

worin
$R^1$ Wasserstoff, $C_1$-$C_8$-Alkylcarbonyl darstellt oder worin die beiden $R^1$-Reste zusammen einen Isopropyliden-Rest darstellen und
$R^2$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und
$R^3$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind oder $R^3$ einen Rest der Formel -CO-X-$R^4$ bedeutet, wobei
X Sauerstoff oder NH und
$R^4$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und $R^4$ für den Fall, daß X NH bedeutet auch ein Rest der Formel --

EP 0 338 435 A1

$(CH_2)_2$-$NR_5R_6$ sein kann, wobei $R^5$ und $R^6$ Wasserstoff oder $C_1$-$C_7$-Alkyl ist oder wobei $R^5$ und $R^6$ zusammen mit dem Stickstoffatom an dem sie gebunden sind einen Pyrrolidin-, Piperidin-, Piperazin- oder Morpholin-Ring bilden, wobei diese Pyrrolidin-, Piperazin-, Piperidin- und Morpholin-Reste ihrerseits gegebenenfalls mit $C_1$-$C_7$-Alkyl mono- oder disubstituiert sind
sowie deren physiologisch verträglichen Salze. Die Verbindungen der Formel (I) zeigen nootrope Wirkung.

# 3,4-Dihydroxypyrrolidin-2-on-Derivate, Verfahren zu ihrer Herstellung diese enthaltende Mittel und ihre Verwendung sowie die bei der Herstellung anfallenden neuen Zwischenprodukten

3-Hydroxypyrrolidin-2-on Derivate sind bekannt (EP-A 0071216). Diese Verbindungen sind nützlich bei der Bekämpfung bzw. Verhütung der cerebralen Insuffizienz bzw. bei der Verbesserung der intellektuellen Leistungsfähigkeit.

Die vorliegende Erfindung betrifft nun:

3,4-Dihydroxypyrrolidin-2-on-Derivate der Formel I

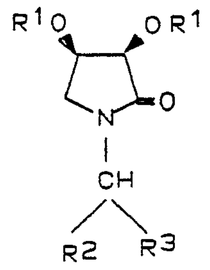

(I)

worin

$R^1$ Wasserstoff, $C_1$-$C_8$-Alkylcarbonyl darstellt oder worin die beiden $R^1$-Reste zusammen einen Isopropyliden-Rest darstellen und

$R^2$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und

$R^3$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind oder $R^3$ einen Rest der Formel -CO-X-$R^4$ bedeutet, wobei

X Sauerstoff oder NH und

$R^4$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und $R^4$ für den Fall, daß X NH bedeutet auch ein Rest der Formel --$(CH_2)_2$-$NR_5R_6$ sein kann, wobei $R^5$ und $R^6$ Wasserstoff oder $C_1$-$C_7$-Alkyl ist oder wobei $R^5$ und $R^6$ zusammen mit dem Stickstoffatom an dem sie gebunden sind einen Pyrrolidin-, Piperidin-, Piperazin- oder Morpholin-Ring bilden, wobei diese Pyrrolidin-, Piperazin-, Piperidin- und Morpholin-Reste ihrerseits gegebenenfalls mit $C_1$-$C_7$-Alkyl mono- oder disubstituiert sind

sowie deren physiologisch verträglichen Salze.

Insbesondere betrifft die Erfindung:

3,4-Dihydroxypyrrolidin-2-on-Derivate der Formel I wie oben angegeben und definiert, dadurch gekennzeichnet, daß mindestens eine der nachfolgenden Bedingungen erfüllt ist $R^1$ bedeutet Wasserstoff

$R^2$ bedeutet Wasserstoff, Phenyl oder Thienyl, wobei die Phenyl- und Thienylreste gegebenenfalls mit $CF_3$, F,

Cl, Br, J, $C_1$-$C_4$-Alkyl, $C_1$-$C_4$-Alkoxy, OH, COOH, COOMe, COOEt, $NO_2$ oder CN mono- oder disubstituiert sind

$R^3$ bedeutet Wasserstoff oder -CO-$NHR^4$ wobei $R^4$ Wasserstoff oder -$(CH_2)_2$-$NR^5R^6$ bedeutet, wobei $R^5$ und $R^6$ $C_1$-$C_3$-Alkyl ist oder wobei $R^5$ und $R^6$ zusammen mit dem Stickstoffatom an dem sie gebunden sind einen Di-$C_1$-$C_3$-Alkyl substituierten Piperidin-Ring bilden.

Weiterhin betrifft die Erfindung ein Verfahren zur Herstellung von 3,4-Dihydroxypyrrolidin-2-on-Derivaten der Formel I, wobei man

a) ein Amin der Formel II

$R^2$ - CH($NH_2$) - $R^3$ (II)

worin $R^2$ und $R^3$ die oben zu Formel I angegebenen Bedeutungen haben mit der Verbindung der Formel III

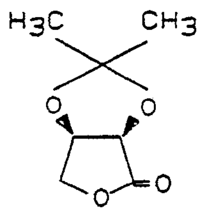

( I I I )

zu Amiden der Formel IV

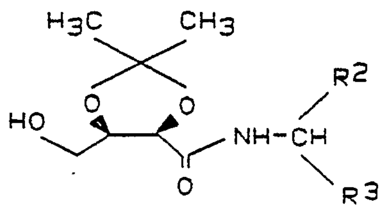

( I V )

worin $R^2$ und $R^3$ die oben zu Formel I angegebenen Bedeutungen haben, umsetzt und anschließend die Amide der Formel IV in eine Verbindung der Formel V

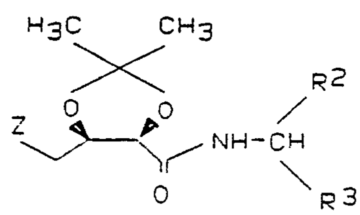

( V )

überführt, worin $R^2$ und $R^3$ die oben zu Formel I angegebenen Bedeutungen haben und Z eine nukleofuge Gruppe ist und dann die Verbindung der Formel V in die geschützten Pyrrolidinon-Derivate der Formel I′ überführt,

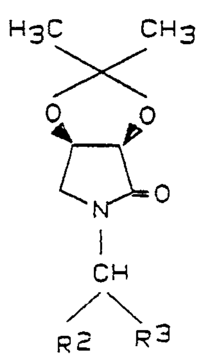

( I′ )

worin $R^2$ und $R^3$ die oben zu Formel I angegebenen Bedeutungen haben oder daß man

b) (3R,4R)-3,4 Di-O-isopropylidenerythronsäurelactam (Formel VI)

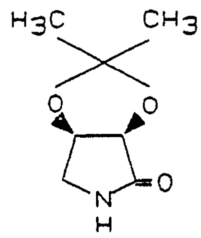

(VI)

mit einer Verbindung der Formel VII

$R^2 - \overset{Z}{\overset{|}{C}}H - R^3$ (VII)

alkyliert, wobei $R^2$ und $R^3$ die oben zu Formel I angegebenen Bedeutungen haben und Z eine nukleofuge Gruppe ist, wobei eine Verbindung der Formel $I'$ entsteht und daß man anschließend in den nach Verfahrensvarianten a) oder b) erhältlichen Verbindungen der Formel $I'$ gegebenenfalls die HydroxySchutzgruppe in 3,4-Position des Pyrrolidinon-Rings abspaltet und die dabei entstandenen 3,4-Dihydroxypyrrolidin-2-one schließlich in die 3,4-Di-O-alkanoyl-pyrrolidin-2-one überführt und diese dann gegebenenfalls noch in ihre physiologisch verträglichen Salze überführt.

Außerdem betrifft die Erfindung die bei der Herstellung der erfindungsgemäßen Verbindungen anfallenden neuen Zwischenprodukte der Formel IV und V.

Unter Aryl werden aromatische Kohlenwasserstoffe, insbesondere Phenyl und Naphthyl verstanden und unter Heteroaryl werden aromatische Kohlenwasserstoffe mit mindestens einem Heteroatom wie N, S oder O verstanden. Beispiele hierfür sind: Furan, Pyrrol, Pyrazol, Imidazol, Pyridin, Pyrimidin, Pyrazin, Indol, Chinolin, Isochinolin, Thiophen. Bevorzugt werden unter Heteroaromaten 2- oder 3-Furyl-, 2- oder 3-Thienyl-, 2- oder 3-Pyrrolyl-, 2- oder 3-Imidazolyl und 2-, 3- oder 4-Pyridyl-Reste verstanden.

Erfindungsgemäß können die Aryl- und Heteroarylreste auch durch pharmakologisch unbedenkliche Reste wie $CF_3$, F, Cl, Br, J, $C_1$-$C_4$-Alkyl, $C_1$-$C_4$-Alkoxy, OH, COOH, COOMe, COOEt, $NO_2$ oder CN ein- oder auch mehrfach substituiert sein.

Alkylreste mit 3 und mehr Kohlenstoffatomen können sowohl geradkettig als auch verzweigt sein.

Unter einer nukleofugen Gruppe werden in der organischen Synthese gängige "Abgangsgruppen" verstanden, wie Cl, Br, J, $OSO_2CH_3$, $OSO_2CF_3$, Phenylsulfonat oder Toluolsulfonat.

Unter Halogen wird Fluor, Chlor, Brom und Jod, insbesondere Chlor, Brom und Jod verstanden.

Im folgenden wird mit "niederem Alkyl" ein $C_1$-$C_7$-Alkylrest bezeichnet.

Die Verbindungen der Formel I enthalten in der 3- und 4-Position des Pyrrolidinon-Rings zwei asymmetrische Kohlenstoffatome, welche beide in der (R)-Konfiguration vorliegen. Verbindungen der Formel I in denen $R^2 \neq$ Wasserstoff und $R^3 \neq$ Wasserstoff sind besitzen mindestens ein weiteres Asymmetriezentrum, wobei das die beiden Reste $R^2$ und $R^3$ tragende Kohlenstoffatom sowohl (S)- als auch (R)-konfiguriert sein kann.

Im folgenden wird die Herstellung von Verbindungen gemäß Formel I und die Herstellung der dafür benötigten Ausgangssubstanzen näher beschrieben.

Verfahrensvariante a) gestattet den Einsatz nahezu beliebiger primärer oder sekundärer Amine der allgemeinen Formel II, wobei für den Fall, daß $R^2$ und $R^3 \neq$ Wasserstoff sind auch optisch aktive Amine, wie z.B. Aminosäureester, unter Beibehaltung der Konfiguration des den Aminostickstoff tragenden Kohlenstoffatoms verwendet werden können und somit letzlich stereochemisch einheitliche Pyrrolidinonderivate der allgemeinen Formel I mit definierter absoluter Konfiguration erhalten werden. Dagegen kommen für die Verfahrensvariante b) bevorzugt nur die reaktiveren Vertreter der allgemeinen Formel VII in Frage, wobei für den Fall, daß sowohl $R^2$ als auch $R^3 \neq$ Wasserstoff sind, zwei diastereomere Alkylierungsprodukte der allgemeinen Formel $I'$ entstehen können. Die nach einer der beiden Verfahrensvarianten a) oder b) hergestellten Verbindungen der Formel $I'$, die noch die 3,4-Di-O-Isopropylidenschutzgruppe enthalten, werden mittels saurer Katalysatoren in die ungeschützten 3,4-Dihydroxyverbindungen (Formel I: $R^1$ = H) überführt.

Verbindungen der allgemeinen Formel I, in denen $R^1$ $C_1$-$C_8$-Alkylcarbonyl bedeutet, werden aus den freien Hydroxyverbindungen durch Umsetzung mit einem einen $C_1$-$C_8$-Alkylcarbonylrest liefernden Mittel hergestellt.

Verbindungen der Formel Ib

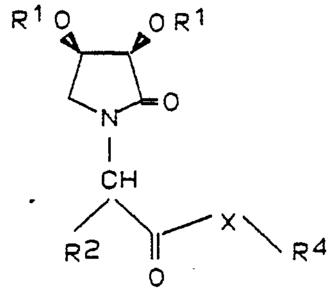

(Ib)

worin R', $R^2$ und $R^4$ die oben zu Formel I angegebenen Bedeutungen haben und X, NH bedeutet, werden bevorzugt aus den nach Verfahrensvarianten a) oder b) erhältlichen isopropylidengeschützten Carbonsäureestern (I:R' = $C(CH_3)_2$, X = 0, $R^4$ = beispielsweise $C_1$-$C_7$-Alkyl oder Benzyl und den Aminen der Formel VIII

$R^4$ -$NH_2$ (VIII)

worin $R^4$ die oben zu Formel I angegebenen Bedeutungen hat, oder aus den durch alkalische Verseifung oder Hydrolyse der Carbonsäureester erhältlichen freien Carbonsäuren durch Peptidverknüpfung über ein anderes reaktionsfähiges funktionelles Derivat mit den betreffenden Aminen der Formel VIII nach bekannten Verfahren erhalten.

Die Herstellung der erfindungsgemäßen Verbindungen nach Verfahrensvariante a) gelingt am einfachsten dadurch, daß man ein Amin oder einen Aminosäureester oder ein Aminosäureamid der allgemeinen Formel II mit dem Lacton der Formel III bei Temperaturen von 0 °C bis 160 °C, je nach Reaktivität der Aminkomponente 30 Minuten bis 14 Stunden in einem inerten organischen Lösungsmittel umsetzt. Primäre Amine der allgemeinen Formel II in denen $R^2$ Wasserstoff bedeutet, reagieren bereits oberhalb 0 °C beim Mischen mit dem Lacton in Substanz oder in Gegenwart eines geeigneten inerten organischen Lösungsmittels innerhalb 10 Minuten bis 10 Stunden quantitativ zu den Amiden der Formel IV. Geeignete Lösungsmittel sind beispielsweise Ether, wie Tetrahydrofuran, Diethylether, tert.-Butylmethylether, Dioxan, Ethylenglykol-dimethylether oder halogenierte Kohlenwasserstoffe wie Methylenchlorid, Chloroform, 1,2-Dichlorethan oder auch Acetonitril oder Dimethylformamid. Man arbeitet dabei zweckmäßig in einem Temperaturbereich von etwa 0 °C bis zur Siedetemperatur des Reaktionsgemisches, zweckmäßigerweise jedoch bei Raumtemperatur. Sekundäre Amine der Formel II in denen $R^2$ und $R^3$ ≠ Wasserstoff sind reagieren bei 20 bis 160 °C in einem der genannten organischen Lösungsmittel innerhalb von 30 Minuten bis 14 Stunden mit dem Lacton der Formel III zu den Produkten der Formel IV. Die von der allgemeinen Formel II abgeleiteten Aminosäureester mit der Bedeutung $R^3$ = $C(O)OR^4$($R^4$ ≠ H) und Aminosäureamide $R^3$ = $C(O)NHR^4$ reagieren mit dem Lacton der Formel III erst bei mehrstündigem Erhitzen (1 Stunden bis 14 Stunden) auf 60 bis 160 °C in einem der genannten, oberhalb 60 °C siedenden Lösungsmittel.

Gemäß Verfahrensvariante a) können Verbindungen der Formel V hergestellt werden, indem die primären Alkohole der Formel IV nach einem der zahlreichen, gängigen Verfahren entweder zu den primären Chloriden, Bromiden oder Jodiden halogeniert (Houben Weyl, Methoden der organischen Chemie, Georg Thieme Verlag, Stuttgart, 1984, Band 6, 1b/III, 927 ff) oder durch Veresterung mit dem betreffenden Sulfonsäurehalogenid oder Sulfonsäureanhydrid, in Gegenwart von Basen, zu den primären Sulfonaten umgesetzt werden (Loc. cit. Houben-weyl). Beispiele für geeignete Halogenierungsreagenzien sind Thionylchlorid, Polyhalogenmethane in Kombination mit Verbindungen des dreiwertigen Phosphors (Z.B. Triphenylphosphan, Phosphorigsäure-tris-(dimethylamid)), Chlortrimethylsilan/Lithiumbromid und Chlortrimethylsilan/Natriumjodid. Ein besonders geeignetes Bromierungsreagenz stellt die Kombination Triphenylphosphan, N-Bromsuccinimid (NBS) dar (Review: B.R. Castro, Org. React. 29, 1 (1983)). So werden in einem bevorzugten Verfahren die Bromide der allgemeinen Formel V (Z = Br) durch portionsweise Zugabe von 1,0-1,3 Äquivalenten NBS unter Eiskühlung zu einer äquimolaren Mischung der Alkohole IV und Triphenylphosphan in einem geeigneten inerten organischen Lösungsmittel, wie Tetrahydrofuran, Diethylether, Ethylenglykoldimethylether und Nachrühren für 1-5 Stunden bei Reaktionstemperaturen um Raumtemperatur in einfacher Weise hergestellt.

Gemäß Verfahrensvariante a) können Pyrrolidinone der Formel I′ aus den Zwischenprodukten der Formel V dadurch hergestellt werden, daß man die Amide V mit einer zur Abstraktion des Protons am Amidstickstoff befähigten Base (z.B. mit Natriumhydrid oder gepulvertem Kaliumhydroxid unter Phasentransfer-Bedingungen) in einem geeigneten inerten organischen Lösungsmittel behandelt. In einer bevorzugten Ausführungsform wird eine Verbindung der Formel V portionsweise zu einer eisgekühlten, gerührten Vorlage einer Natriumhydridsuspension in Tetrahydrofuran, Diethylether, Dioxan oder Dimethylformamid gegeben. Je nach Reaktivität des verwendeten Halogenderivates bzw. des eingesetzten Sulfonates läßt man die Mischung noch 0,5-12 Stunden bei 0° C bis zur Siedetemperatur des Lösungsmittels reagieren. Verwendet man gepulvertes Natrium- oder Kaliumhydroxid als Base, so setzt man der Reaktionsmischung ca. 0,01-0,1 Moläquivalente eines Phasentransferkatalysators wie beispielsweise tetra-n-Butylammoniumbromid- oder -jodid oder Benzyltriethylammoniumchlorid oder -bromid zu.

Gemäß Verfahrensvariante b) können Verbindungen der Formel I′ dadurch hergestellt werden, daß man das geschützte Pyrrolidinon der Formel VI mit einer zur Deprotonierung am Amidstickstoff befähigten Base (z.B. mit Natrium-/Kaliumhydrid) behandelt und hierauf das erhaltene Anion mit dem reaktiven Alkylierungsreagenz der allgemeinen Formel VII versetzt. In einer bevorzugten Variante kommt ein "Eintopfverfahren" zur Alkylierung von Lactamen unter Fest-Flüssig-Phasentransferkatalyse zur Anwendung (Tetrahedron-Lett. (1978), 615; Synthesis 527, 1979), bei dem eine Mischung des Pyrrolidinons VI, des Alkylierungsreagenzes VII, eines Phasentransferkatalysators wie z.B. Tetra-n-butylammoniumbromid und gepulvertem Kaliumhydroxid, in Tetrahydrofuran oder Diethylether bei Temperaturen um Raumtemperatur umgesetzt wird. Als Base eignet sich auch gepulvertes Natriumhydroxid und als Phasentransferkatalysator können beispielsweise auch Benzyltriethylammoniumchlorid, -bromid, oder jodid verwendet werden.

Die gemäß den Verfahrensvarianten a) oder b) erhältlichen geschützten Pyrrolidinone I′ können durch Abspaltung der Isopropylidenschutzgruppe in die Verbindungen der Formel I überführt werden. Die Entfernung der Schutzgruppe erfolgt nach an sich bekannten Methoden, beispielsweise durch Behandeln mit verdünnter Salzsäure in Tetrahydrofuran, mit Chlorwasserstoff in Methanol oder Ethanol, durch Umacetalisieren mit einem niederen Alkohol, wie Methanol oder Ethanol, in Gegenwart eines sauren Katalysators, wie p-Toluolsulfonsäure oder sauren Ionenaustauschern. Dabei können andere im Molekül vorhandene Schutzgruppen mit abgespalten werden, wie z.B. die tert.-Butylestergruppe in Verbindungen der Formel Ib), bei denen $XR^4$ die Bedeutung $O\text{-}C(CH_3)_3$ hat.

Verbindungen der allgemeinen Formel Ib worin X die Bedeutung NH besitzt, können einerseits nach den oben beschriebenen Verfahrensvarianten a) und b) hergestellt werden und andererseits, indem die nach beiden Verfahren zugänglichen Verbindungen der allgemeinen Formel Ib mit der Bedeutung X=O über die freien Carbonsäuren mit $XR^4 = OH$ oder reaktionsfähige funktionelle Derivate derselben, mit Aminen der allgemeinen Formel VIII in die betreffenden Amide umgewandelt werden. Verwendet man die freie Carbonsäure der Formel Ib ($XR^4$ = OH) in Gegenwart eines reaktiven Kondensationsmittels oder reaktive funktionelle Derivate davon, wie entsprechende Carbonsäurehalogenide oder -anhydride, so kommen als Ausgangsstoffe nur solche Verbindungen in Frage, worin $R^1$ nicht Wasserstoff bedeutet. Erzeugt man die genannten freien Carbonsäuren beispielsweise durch sauer katalysierte Verseifung der entsprechenden tert.-Butylester der Formel Ib ($XR^4 = OC(CH_3)_3$), so werden durch gleichzeitige Abspaltung der Isopropylidenschutzgruppe die freien Dihydroxyverbindungen erzeugt. Für die Herstellung freier Carbonsäuren der Formel Ib ($XR^4$ = OH), mit intakter Isopropylidenschutzgruppe kommen als Ausgangsstoffe deshalb insbesondere die niederen Alkyl bzw. die Benzylester zur Anwendung, weil diese nach Standardmethoden der organischen Synthese, wie alkalische Verseifung bzw. Hydrogenolyse, unter Erhalt der Isopropylidenschutzgruppe abspaltbar sind.

Für die Kondensation der freien Carbonsäuren der Formel Ib) ($XR^4$ = OH) mit Aminen der Formel VIII eignen sich als Kondensationsmittel beispielsweise Dicyclohexylcarbodiimid, gegebenenfalls zusammen mit N-Hydroxysuccinimid oder 1-(niedere Alkyl)-2-halogen-pyridiniumsalze. Man arbeitet dabei in einem Temperaturbereich von etwa 0° C bis zum Siedepunkt des Reaktionsgemisches, zweckmäßigerweise jedoch bei Raumtemperatur und verwendet geeignete inerte organische Lösungsmittel wie Ether, beispielsweise Tetrahydrofuran, Diethylether, tert.-Butylmethylether, Dioxan, Ethylenglykol-dimethylether oder halogenierte Kohlenwasserstoffe, wie Methylenchlorid, Chloroform, 1,2-Dichlorethan oder Acetonitril oder Dimethylformamid.

Verwendet man ein reaktionsfähiges funktionelles Derivat einer isopropylidengeschützten Carbonsäure der Formel Ib ($XR^4$ = OH, $R^1 = C(CH_3)_2$), so kommen neben den genannten niederen Alkylestern entsprechende Carbonsäurehalogenide, insbesondere die Carbonsäurechloride, entsprechende Carbonsäureanhydride und gemischte Anhydride (z.B. mit Mesitylen-sulfonsäure oder Ameisensäureethylester) oder entsprechende Carbonsäureimidazolide in Betracht. Die reaktionsfähigen funktionellen Derivate der Formel Ib müssen nicht in allen Fällen isoliert, sondern können in situ hergestellt und unmittelbar weiterverarbeitet werden. Zweckmäßigerweise arbeitet man in einem der oben genannten inerten organischen Lösungsmitteln und je nach Reaktivität des verwendeten Carbonsäurederivates in einem Temperaturbereich von etwa 0° C bis zum Siedepunkt des Reaktionsgemisches.

In einer bevorzugten Ausführungsform verwendet man einen niederen Alkylester einer isopropylidengeschützten Carbonsäure der Formel Ib (X=O, $R^1 = C(CH_3)_2$) und überschüssiges Amin der Formel VIII als Lösungsmittel. Soll ein am Stickstoff unsubstituiertes Amid der Formel Ib (X = NH, $R^4$ = H) hergestellt

werden, so handelt es sich bei der Verbindung der Formel VIII um Ammoniak, welcher vorzugsweise in wäßriger oder alkoholischer, insbesondere methanolischer, Lösung eingesetzt wird.

Verbindungen der Formel I und Ib, worin $R^1$ $C_1$-$C_8$-Alkanoyl bedeutet, können dadurch hergestellt werden, daß man eine Verbindung der Formel I bzw. Ib, worin $R^1$ Wasserstoff ist und, $R^2$, $R^3$ bzw. $XR^4$ die angebenenen Bedeutungen besitzen, mit einem einen $C_1$-$C_8$-Alkanoylrest lieferenden Mittel behandelt. Geeignete, einen $C_1$-$C_8$-Alkanoylrest liefernde Mittel sind beispielsweise niedere Alkancarbonsäurehalogenide, insbesondere die Chloride, entsprechende Anhydride und gemischte Anhydride oder entsprechende niedere Alkancarbonsäureimidazolide. Diese Verbindungen sind in den meisten Fällen käuflich. Zweckmäßigerweise arbeitet man in einem inerten organischen Lösungsmittel, wobei insbesondere Ether, wie Tetrahydrofuran, Diethylether, t-Butylmethylether, Dioxan, Ethylenglykoldimethylether oder halogenierte Kohlenwasserstoffe wie Methylenchlorid, Chloroform, 1,2-Dichlorethan oder aromatische Kohlenwasserstoffe, wie Toluol oder auch Acetonitril oder Dimethylformamid in Frage kommen. Die Reaktionstemperatur liegt zweckmäßigerweise in einem Bereich von etwa 0 °C bis zur Siedetemperatur des Reaktionsgemisches.

Die Verbindungen der Formel II sind käuflich oder lassen sich nach Standard-Verfahren der organischen Synthese aus käuflichen Vorstufen herstellen. Verfahren zur Herstellung von primären Aminen der Formel II finden sich in: Houben-Weyl Bd. XI/1 (1957) und Bd. E5/1 (1985), G. Thieme Verlag, Stuttgart.

Die Verbindung III kann in großem Maßstab aus der käuflichen D(-)-Isoascorbinsäure nach N. Cohen et al., J. Am. Chem. Soc. 105, 3661 (1983) erhalten werden.

Die Verbindung VI wird aus der Verbindung III nach S. Hanessian, J. Org. Chem. 34, 675 (1969) über die Zwischenstufen IX und X erhalten.

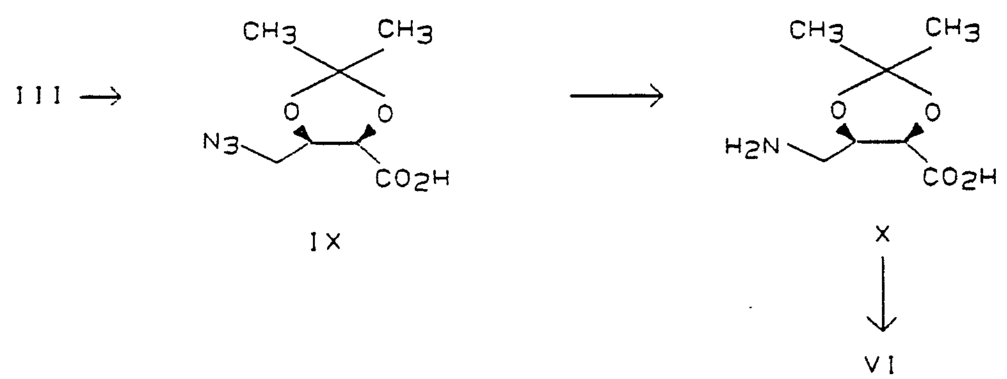
III, IX, X, VI

Anstelle der von S. Hanessian im 100 mg Maßstab mittels Vakuumsublimation von X beschriebenen Herstellung von VI in 80 % Ausbeute, wird dieser Reaktionsschritt durch einfache Azeotropdestillation von X mit Toluol am Wasserabscheider im 100 g-Maßstab und in quantitativer Ausbeute durchgeführt. Das so erhaltene Lactam fällt kristallin und in höherer Reinheit (höherer Drehwert) als beim Literaturverfahren an.

Die Verbindungen der Formel VII sind, sofern nicht käuflich, z.B. nach Houben-weyl Bd. 6/1b, S. 927 ff., G. Thieme Verlag, Stuttgart (1984) darstellbar.

Die Herstellung von pharmazeutisch akzeptabelen Basen- oder Säureadditionsalzen von Verbindungen der Formel I erfolgt nach allgemein üblichen und jedem Fachmann geläufigen Methoden. Für basische Verbindungen kommen sowohl Salze mit anorganischen als auch Salze mit organischen Säuren in Betracht, beispielsweise Hydrochloride, Hydrobromide, Sulfate, Methansulfonate, p-Toluolsulfonate, Oxalate, Tartrate, Citrate, Maleinate, Ascorbate oder Acetate. Für saure Verbindungen sind insbesondere geeignet Alkali- und Erdalkalihydroxide, Carbonate oder Bicarbonate sowie physiologisch verträgliche organische Verbindungen, die eine primäre, sekundäre oder tertiäre Aminogruppe tragen, wie Ammonium-, Pyridinium- oder Triethylammoniumsalze.

Die Verbindungen der Formel I besitzen wertvolle pharmakologische Eigenschaften, insbesondere nootrope. Die nootrope Wirkung der erfindungsgemäßen Verbindungen wurde an Mäusen, die ein Körpergewicht von 20-25 g besaßen, im inhibitory (passive) avoidance test (step-through-test) geprüft. Eine modifizierte Form der von J. Kopp, Z. Bodanecky und M.E. Jarvik beschriebenen Test-Methode wurde von J. Bures, O. Buresova und J. Huston im "Techniques and Basic Experiments for the Study of Brain and Behavior", Elsevier Scientific Publishers, Amsterdam 1983, beschrieben.

Entsprechend diesen Literaturangaben wird eine Substanz dann als nootrop wirksam bezeichnet, wenn sie bei den Versuchstieren die mittels eines electroconvulsiven Schocks erzeugte Amnesie (ECS-Amnesie) bzw. mittels Scopolamin-Injektion erzeugte Amnesie aufzuheben vermag.

Die Versuche wurden nach modifizierten Test-Methoden mit den erfindungsgemäßen Verbindungen durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß der amnestische Effekt bereits mit einer MED (minimal effective dose) von 1-10 mg/kg p.o. aufzuheben ist. Die Vergleichssubstanz hat eine MED von ca. 500-1000 mg/kg p.o.

In Tabelle 1 und 2 sind die MED für einige erfindungsgemäße Verbindungen aufgeführt.

Tabelle 1

| erfindungsgemäße Verbindung aus Beispiel | Inhibitory avoidance MED mg/kg p.o. ECS-Amnesie |
|---|---|
| 29 | 3 |
| 28 | 10 |
| 33 | 3 |
| 34 | 3 |
| 36 | 3 |
| Piracetam Vgl. Beispiel | 1000 |

Tabelle 2

| erfindungsgemäße Verbindung aus Beispiel | Inhibitory avoidance MED mg/kg p.o. Scopolamin-Amnesie |
|---|---|
| 29 | 1 |
| 34 | 1 |
| Piracetam Vgl. Beispiel | 500 |

Die erfindungsgemäßen Verbindungen sind auf Grund ihrer pharmakologischen Eigenschaften für die Behandlung cognitiver Dysfunktionen unterschiedlicher Genese, wie sie z.B. bei der Alzheimer'schen Krankheit oder der Senilen Demenz auftreten geeignet.

Die Erfindung betrifft daher weiter die Anwendung der erfindungsgemäßen Verbindungen bei der Behandlung und Prophylaxe cognitiver Dysfunktionen.

Die neuen Verbindungen können entweder allein oder mit physiologisch verträglichen Hilfs- oder Trägerstoffen vermischt als Arzneimittel angewandt werden. Sie können zu diesem Zweck oral in Dosen von 0,01-30,0 mg/kg/Tag, vorzugsweise 0,01-10,0 mg/kg/Tag, insbesondere 0,05-2,0 mg/kg/Tag oder parenteral (z.B. intravenös, subkutan oder intramuskulär) in Dosen von 0,001 3,0 mg/kg/Tag, vorzugsweise 0,001-1,0 mg/kg/Tag, insbesondere 0,005-0,2 mg/kg/Tag appliziert werden. Die Dosierung kann in schweren Fällen auch erhöht werden. In vielen Fällen genügen jedoch auch geringere Dosen.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral, intravenös oder rectal appliziert werden, wobei die orale Applikation

bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige, alkoholische oder ölige Suspensionen oder wäßrige oder ölige Lösungen. Als inerte Trägerstoffe können z.B. Gummi arabicum, Magnesia, Magnesiumcarbonat, Kaliumphosphat, Milchzucker, Glukose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z.B. in Frage physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

**Beispiel 1**

Herstellung von

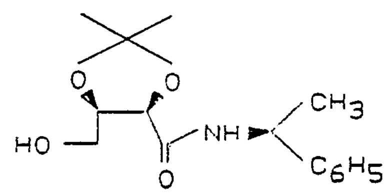

6.64 g (42 mmol) 3R,4R-O-isopropyliden-erythronsäurelacton (III) und 6,06 g (50 mmol) (S)-1-phenylethylamin werden in 20 ml wasserfreiem Dichlormethan 8 h bei 40 °C erhitzt. Nach Waschen mit 20 ml 0,5 N Salzsäure, Trocknen über Natriumsulfat und Einengen erhält man 11,0 g (94 %) 2R,3R-O-isopropyliden-erythronsäure-(S)-1-phenylethylamid als farbloses Öl, das direkt in der folgenden Stufe eingesetzt werden kann.

**Beispiel 2**

Herstellung von

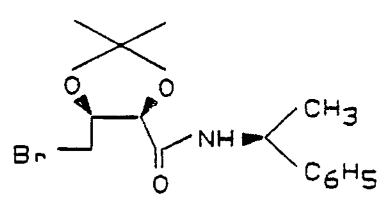

41, 1 g (147 mmol) Produkt aus Beispiel 1 und 38,6 g (147 mmol) Triphenylphosphin werden in 200 ml Tetrahydrofuran (THF) gelöst und bei 0 °C unter Rühren portionsweise mit 29,4 g (165 mmol) N-Bromsuccinimid versetzt. Nach beendeter Zugabe rührt man noch 1 h bei Raumtemperatur. Nach Abdestillieren des THF wird der Rückstand in 500 ml Diisopropylether verrührt und filtriert. Das Filtrat wird nach Waschen mit gesättigter Natriumhydrogencarbonatlösung getrocknet und eingeengt. Filtration über Kieselgel mit Diisopropylether liefert 45,8 g (91 %) 4-Brom-4-deoxy2R,3R-O-isopropyliden-erythronsäure-(S)-1-phenylethylamid als farbloses viskoses Öl, das für die folgende Umsetzung genügend rein ist (Dünnschichtchromatogramm).

**Beispiel 3**

Herstellung von

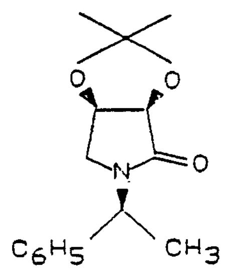

45,8 g (134 mmol) Produkt aus Beispiel 2 in 100 ml wasserfreiem THF werden unter Eiskühlung und Rühren zu einer Suspension von 150 mmol Natriumhydrid und 2 g Kaliumjodid in 100 ml THF getropft. Man rührt noch 1 h bei Raumtemperatur nach, engt ein, nimmt den Rückstand in Dichlormethan auf und wäscht die organische Phase mit einer gesättigten Ammoniumchloridlösung. Nach Trocknen ($Na_2SO_4$) und Einengen wird der Rückstand aus Diisopropylether:Hexan umkristallisiert. Die Ausbeute an 1-[(S)-1-phenylethyl]-(3R,4R)-3,4-dihydroxy-3,4-di-O-isopropyliden-pyrrolidin-2-on vom Schmelzpunkt 125-126° C beträgt 21,3 g (61 %).
$[\alpha]_D^{20}$ - 129° (C = 1,0, $CH_3OH$).

**Beispiel 4**

Herstellung von

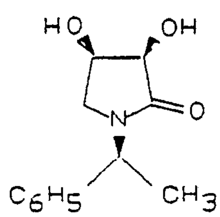

13,4 g (51,3 mmol) Produkt aus Beispiel 3 werden in 40 ml Methanol und 20 ml konz. Salzsäure gelöst. Nach 4 h Stehenlassen bei Raumtemperatur wird zur Trockne eingeengt, mit Essigester über eine Schicht Kieselgel filtriert und das sirupöse Produkt aus Wasser gefriergetrocknet. Ausbeute 10,4 g (92 %) 1-[(S)-1-phenylethyl]-(3R,4R)-3,4-dihydroxypyrrolidin-2-on als farbloses Pulver mit
$[\alpha]_D^{20}$ - 134° (C = 1, $CH_3OH$).

**Beispiel 5**

Herstellung von

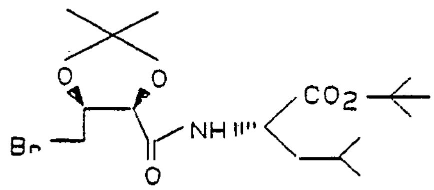

Eine Lösung von 6,64 g (42 mmol) 3R,4R-O-isopropylidenerythronsäurelacton (III) und 11,24 g (60 mmol) L-Leucin-tert.-butylester in 20 ml wasserfreiem Dichlormethan wird 12 h unter Rückfluß erhitzt. Nach Verdünnen mit 300 ml Diethylether wird überschüssiges Amin durch Waschen mit einer 5 proz. Citronensäurelösung entfernt. Trocknung über $Na_2SO_4$ und Einengen gibt 14 g (96 %) N-[(2R,3R)-2,3,4-Trihydroxy-2,3-di-O-isopropyliden-butanoyl]-L-leucintert.-butylester als farbloses Öl, das direkt weiter umgesetzt wird.

**Beispiel 6**

Herstellung von

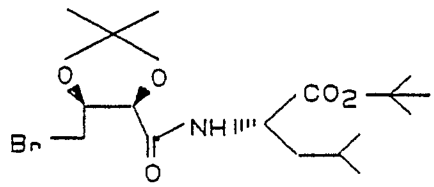

48,4 g (140 mmol) Produkt aus Beispiel 5 und 36,8 g (140 mmol) Triphenylphosphin in 200 ml THF werden bei 0 °C unter Rühren portionsweise mit 29,4 g (165 mmol) N-Bromsuccinimid versetzt. Nach beendeter Zugabe wird noch 1 h bei Raumtemperatur nachgerührt. Nach Einengen wird der Rückstand in 400 ml Diisopropylether verrührt und von den unlöslichen Nebenprodukten abfiltriert. Das Filtrat wird nach Waschen mit gesättigter Natriumhydrogencarbonatlösung getrocknet, eingeengt und über Kieselgel mit Diisopropylether als Laufmittel filtriert. Man erhält 45,8 g (80 %) N-[(2R,3R)-4-Brom-2,3-dihydroxy-2,3-di-O-isopropyliden-butanoyl]-L-leucin-tert.-butylester als farbloses Öl, das für die weitere Umsetzung genügend rein ist.

**Beispiel 7**

Herstellung von

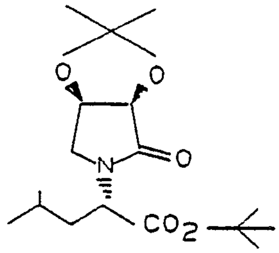

45,8 g (112 mmol) Rohprodukt aus Beispiel 6 in 100 ml THF werden bei 0 °C unter Rühren zu einer Suspension von 120 mmol Natriumhydrid und 2 g Kaliumiodid in 100 ml THF getropft. Nach 1 h Rühren bei Raumtemperatur engt man ein, nimmt den Rückstand in Dichlormethan auf, wäscht die organische Phase

mit gesättigter Ammoniumchloridlösung, trocknet ($Na_2SO_4$) und konzentriert die Lösung zu einem Öl. Aus Diisopropylether/Hexan kristallisieren: 28,5 g (77,6 %) (2S)-4-methyl-2-[(3R,4R)-3,4-dihydroxy-3,4-Di-O-isopropyliden-pyrrolidin-2-on-l-yl]-pentansäure-tert.-butylester vom Schmelzpunkt 90-91 °C.

**Beispiel 8**

Herstellung von

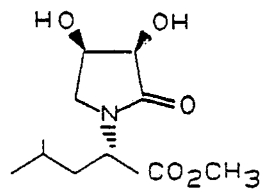

23,0 g (70 mmol) Produkt aus Beispiel 7 werden in 200 ml einer gesättigten methanolischen Chlorwasserstofflösung gelöst. Nach 5 h bei Raumtemperatur engt man zur Trockne ein, nimmt den Rückstand in 200 ml Dichlormethan auf, filtriert und engt das Filtrat erneut ein. Dabei kristallisieren 16,9 g (98 %) (2S)-4-methyl-2-[(3R,4R)-3,4-dihydroxy-pyrrolidin-2-on-1-yl]-pentansäuremethylester aus. Fp. 125-126 °C $[\alpha]_D^{20}$ - 16,3° (C = 1, $CH_3OH$)

**Beispiel 9**

Herstellung von

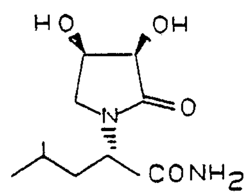

10,0 g (40,8 mmol) Produkt aus Beispiel 8 werden in 200 ml einer gesättigten Lösung von Ammoniak in Methanol aufgelöst. Nach 48 h engt man die Lösung ein, filtriert den Rückstand über eine Schicht Kieselgel mit Dichlormethan/Methanol (10/1) als Laufmittel, engt zur Trockne ein und gefriertrocknet den Rückstand aus Wasser. Beim Versetzen mit Ether kristallisiert das (2S)-4-methyl-2-[(3R,4R)-3,4-dihydroxy pyrrolidin-2-on-1-yl]-pentansäureamid aus. Fp. 163-164 °C
$[\alpha]_D^{20}$ - 108° (C = 1, $CH_3OH$)

**Beispiel 10**

Herstellung von

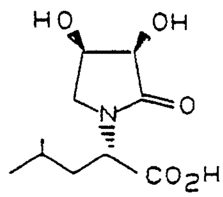

2,30 g (7 0 mmol) Produkt aus Beispiel 7 werden in einer Mischung aus 5 ml Methanol, 20 ml THF und 20 ml 2 N Salzsäure gelöst. Nach 3 h Rühren bei Raumtemperatur engt man zur Trockne ein und kristallisiert das Produkt aus Ethylacetat/Methanol um. Man erhält so 1,20 g (74 %) (2S)-4-methyl 2-[-(3R,4R)-3,4-dihydroxy-pyrrolidin-2-on-1-yl]-pentansäure in farblosen Kristallen vom Schmelzpunkt 158-160° C.

**Beispiel 11**

Herstellung von

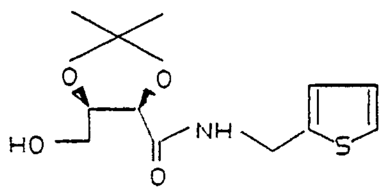

4.98 g (44 mmol) 2-(Aminomethyl)thiophen und 6,64 g (42 mmol) 3R,4R-O-isopropylidenerythronsäure-lacton (III) werden unter leichtem Erwärmen in möglichst wenig Dichlormethan (ca. 15 ml) aufgelöst. Innerhalb von 12 Stunden kristallisiert das Produkt teilweise aus. Man vervollständigt die Kristallisation durch Zugabe von 100 ml Diisopropylether und erhält so 11,1 g (93 %) 2R,3R-O-Isopropylidenerythronsäure-(2-thienylmethyl)-amid vom Schmelzpunkt 97-98° C.

**Beispiel 12**

Herstellung von

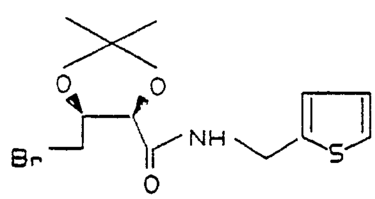

2,71 g (10 mmol) Produkt aus Beispiel 11 und 2,88 g (11 mmol) Triphenylphosphin in 15 ml wasserfreiem THF werden bei 0° C vorgelegt und unter Rühren portionsweise mit 2,05 g (11,5 mmol) N-Bromsuccinimid versetzt. Nach beendeter Zugabe rührt man die Mischung noch I h bei Raumtemperatur, engt ein und nimmt den Rückstand in ca. 100 ml Diisopropylether auf. Unlösliche Bestandteile werden abfiltriert, die Lösung mit gesättigter Natriumhydrogencarbonatlösung gewaschen, getrocknet ($Na_2SO_4$) und eingeengt. Nach Säulenfiltration über Kieselgel mit Diisopropylether erhält man 2,7 g (81 %) 4-Brom-4-deoxy 2R,3R-O-isopropyliden-erythronsäure-(2-thienylmethyl)amid als farbloses Öl, das für die nachfolgende Umsetzung genügend rein ist (Dünnschichtchromatogramm).

**Beispiel 13**

Herstellung von

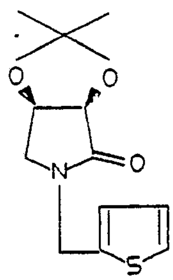

2,70 g (8,1 mmol) Produkt aus Beispiel 12 werden in 20 ml wasserfreiem THF gelöst und unter Eiskühlung mit 330 mg (ca. 11 mmol einer 80 proz. Natriumhydridsuspension in Mineralöl und 500 mg Kaliumiodid versetzt. Man rührt die Mischung noch 1 h bei Raumtemperatur, destilliert das THF ab, nimmt den Rückstand in Dichlormethan auf, wäscht die Lösung mit einer gesättigten wässrigen Ammoniumchloridlösung, trocknet ($Na_2SO_4$), engt ein und kristallisiert das Produkt aus Diisopropylether um. Man erhält so 1,86 g (91 %) (3R,4R)-3,4-Dihydroxy-3,4-di-O-isopropyliden-1-(2-thienylmethyl-pyrrolidin-2-on vom Schmelzpunkt 78-79° C.

**Beispiel 14**

Herstellung von

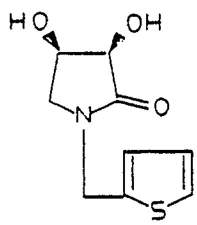

12,0 g (47,3 mmol) Produkt aus Beispiel 13 werden in einer Mischung aus 40 ml Methanol und 20 ml konz. Salzsäure gelöst. Nach 3-stündigem Rühren bei Raumtemperatur engt man zur Trockne ein und kristallisiert das Produkt aus Ethylacetat um. Man erhält so 8,80 g (87 %) (3R,4R)-3,4-Dihydroxy-1-(2 thienylmethyl)-pyrrolidin-2-on vom Schmelzpunkt 103-105° C
$[\alpha]_D^{20}$ - 11,2° (C = 1, $CH_3OH$).

**Beispiel 15**

Herstellung von

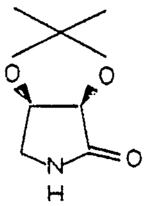

VI

86,0 g (0,49 mol) 4-Amino-4-deoxy-2,3-O-isopropyliden-D-erythronsäure (X) werden mit 500 ml Toluol am Wasserabscheider 6-8 Stunden unter Rückfluß erhitzt, bis kein Toluol/Wasser-Azeotrop mehr übergeht. Nach Einengen und Kristallisation aus Ethylacetat/Ether erhält man 76,2 g (99 %) 4-Amino-4-deoxy-2,3-O-isopropyliden-D-erythronsäurelactam (VI) in farblosen Kristallen vom Schmelzpunkt 148-149 °C und dem Drehwert
$[\alpha]_D^{20}$ - 78,4° (C = 0,78, $CH_3OH$).

S. Hanessian, J. Org. Chem. 34, 675 (1969) erhielt ein Produkt mit dem Drehwert
$[\alpha]_D^{20}$ - 59° (C = 0,78, $CH_3OH$)

**Beispiel 16**

Herstellung von

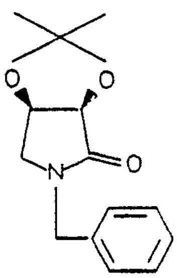

1,57 g (10,0 mmol) Lactam aus Beispiel 15, 0,33 g (1,00 mmol) Tetra-n-butylammoniumbromid und 2,23 g (13 mmol) Benzylbromid werden in 20 ml wasserfreiem THF vorgelegt und unter Eiskühlung portionsweise mit 0,73 g (13 mmol) fein gepulvertem Kaliumhydroxid versetzt. Dann rührt man noch 3 h bei Raumtemperatur, gibt 200 ml Ethylacetat zu und wäscht die organische Phase mehrfach mit Wasser. Nach Trocknen ($Na_2SO_4$) und Einengen kristallisiert das Produkt bei Zugabe von Ether/Hexan aus. Man erhält 2,46 g (99 %) 1-Benzyl-(3R,4R)-3,4-dihydroxy-3,4-di-O-isopropyliden-pyrrolidin2 on vom Schmelzpunkt 85-86 °C und mit dem Drehwert
$[\alpha]_D^{20}$ + 6,7° (C = 0,2, $CH_3OH$).

**Beispiel 17**

Herstellung von

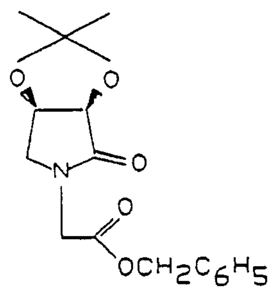

1,57 g (10,0 mmol) Lactam aus Beispiel 15 und 300 mg (ca. 12 mmol) einer 80 proz. Natriumhydridsuspension in Mineralöl werden bei Eisbadtemperatur 20 min. in 15 ml wasserfreiem N,N-Dimethylformamid (DMF) gerührt. Nach Zugabe von 2,52 g (11,0 mmol) 2 Bromessigsäurebenzylester rührt man die Mischung noch 1 h bei Raumtemperatur, gibt 50 ml einer gesättigten wässrigen Ammoniumchloridlösung zu und extrahiert 3 mal mit je 100 ml Dichlormethan. Nach Trocknen ($Na_2SO_4$) und Einengen wird das Produkt mit n-Hexan digeriert. Dabei erhält man 2,59 g (85 %) kristallinen 2-[(3R,4R)-3,4-Dihydroxy-3,4-di-O-isopropylidenpyrrolidin-2-on-1-yl]essigsäure-benzylester, der bei 50-52 °C schmilzt.

Die Produkte der Beispiele 18-24 werden analog den Beispielen 16 und 17 dargestellt und sind mit Angabe der Ausbeuten und ausgewählten physikalischen Daten in Tabelle 3 aufgelistet:

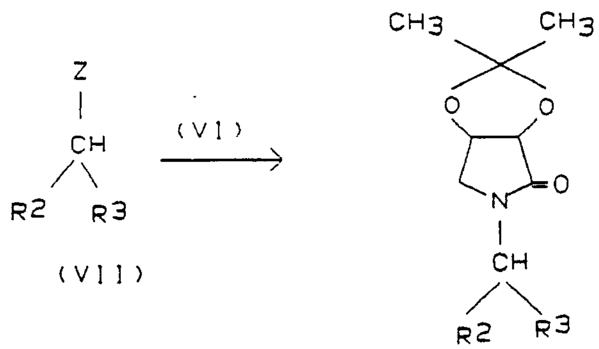

(I′)

Tabelle 3

| Beispiel | $R^2$ | $R^3$ | Z | Verfahren analog Beispiel Nr. | Schmelzpunkt (°C) | Analyse (%) | | | Ausbeute % |
|---|---|---|---|---|---|---|---|---|---|
| | | | | | | C(ber.) | H(ber.) | N(ber.) | |
| | | | | | | C(gef.) | H(gef.) | N(gef.) | |
| 18 | H | $4-NO_2-C_6H_4CH_2$ | Br | 16 | 80-81 | 57,5<br>57,2 | 5,5<br>5,5 | 9,6<br>9,7 | 86 |
| 19 | H | $4-Br-C_6H_4CH_2$ | Br | 16 | 102-104 | 51,5<br>51,4 | 4,9<br>4,8 | 4,3<br>4,2 | 96 |
| 20 | H | $4-F-C_6H_4CH_2$ | Br | 16 | 80-82 | 63,4<br>63,2 | 6,0<br>6,2 | 5,3<br>5,3 | 99 |
| 21 | H | $4-(CH_3)-C_6H_4CH_2$ | Br | 16 | 108-110 | 68,9<br>68,6 | 7,3<br>7,5 | 5,3<br>5,0 | 95 |
| 22 | H | $3-(OCH_3)-C_6H_4CH_2$ | Cl | 16 | Öl | 64,9<br>64,6 | 6,9<br>6,8 | 5,0<br>4,9 | 98 |
| 23 | H | $CONH_2$ | Cl | 17 | 135-137 | 50,4<br>50,4 | 6,6<br>6,8 | 13,0<br>12,8 | 75 |
| 24 | H | $CO_2CH_3$ | Br | 17 | Öl | 52,4<br>52,0 | 6,6<br>6,8 | 6,1<br>5,9 | 82 |

**Beispiel 25**

Herstellung von

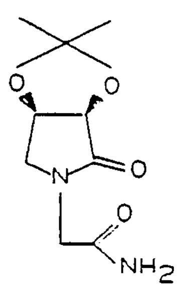

2,29 g (10,0 mmol) 2-[(3R,4R)-3,4-Dihydroxy-3,4-di-O-isopropyliden-pyrrolidin-2-on-1-yl]-essigsäure-methylester (Beispiel 24) werden mit 50 ml gesättigter methanolischer Ammoniaklösung 28 h bei Raumtemperatur umgesetzt. Nach Einengen zur Trockne wird der Rückstand mit Ether verrührt. Dabei kristallisieren 1,92 g (90 %) 2-[(3R,4R)-3,4-Dihydroxy-3,4-di-O-isopropyliden-pyrrolidin-2 on-1-yl]acetamid (siehe Beispiel 23, Tabelle 3) aus.

**Beispiel 26**

Herstellung von

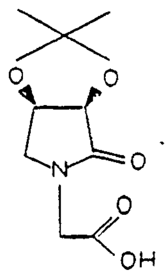

0,5 g (100 mmol) Produkt aus Beispiel 17 werden bei Raumtemperatur und Normaldruck in 200 ml Methanol in Gegenwart von 2 g 10 proz. Palladiumkohle hydriert. Nach Filtration und Eingengen wird der Rückstand aus Diisopropylether verrührt. Dabei erhält man 20,7 g (96 %) -[(3R,4R) 3,4-Dihydroxy-3,4-di-0-isopropyliden-pyrrolidin-on-1-yl]essigsäure vom Schmelzpunkt 96-99 °C.

**Beispiel 27**

Herstellung von

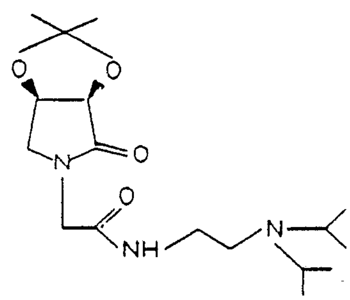

2,29 g (10,0 mmol) 2-[(3R,4R)-3,4-Dihydroxy-3,4-di-O-isopropyliden-pyrrolidin-2-on-1-yl]-essigsäure-methylester werden mit 1,60 g (11,1 mmol) 2-(Diisopropylamino)ethylamin 3 h unter Stickstoff auf 100 °C erwärmt. Nach Zugabe von 200 ml Diethylether entfernt man überschüssiges Amin durch zweimaliges Waschen mit je 10 ml Wasser, trocknet die Etherphase mit $Na_2SO_4$ und engt sie bis zur Trockne ein. Man erhält 2,90 g (84 %) öliges N-[2-(Diisopropylamino) ethyl]-2-[(3R,4R)-3,4-dihydroxy-3,4-di-O-isopropyliden pyrrolidin-2-on-1-yl]-acetamid, das im Dünnschichtchromatogramm im Laufmittelsystem Ethylacetat/Diisopropylether/Methanol, konz. Ammoniak (10/6/3/1) an Kieselgel mit $R_f$ = 0,6 eluiert wird. Es ist genügend rein zur Umsetzung nach Beispiel 28.

**Beispiel 28**

Herstellung von

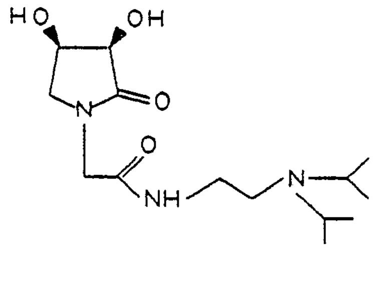

11.0 g (32,2 mmol) Produkt aus Beispiel 27 werden 3 h bei Raumtemperatur mit einer Mischung aus 100 ml Methanol und 10 ml konz. Salzsäure gerührt. Man engt zur Trockne ein und löst den Rückstand in 10 ml einer 3,5 N Kaliumhydroxidlösung, engt wieder vollständig zur Trockne ein, verdünnt den Rückstand mit 100 ml Ethylacetat, filtriert über Celite und destilliert das Lösungsmittel vollständig ab. Nach Säulenchromatographie an Kieselgel mit Ethylacetat/Diisopropylether/Methanol/konz. Ammoniak (10/6/3/1) erhält man 9,24 g (95 %) N-[2-(Diisopropylamino)ethyl]-2-[(3R,4R)-3,4-dihydroxypyrrolidin-2-on-1-yl]acetamid als viskoses Öl.

100 MHz - $^1$H-NMR in D20 ($\delta$-Werte in ppm):

1.28 (d,12H), 2,8-3,9 (m,8H), 4,10 (m,2H,Acetamid-$CH_2$), 4,5 (m,2H,CHO).

**Beispiel 29**

Herstellung von

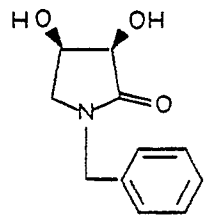

8.50 g (34 mmol) Produkt aus Beispiel 16 werden in einer Mischung aus 100 ml THF, 50 ml Methanol und 5 ml konz. Salzsäure gelöst und 12 h bei Raumtemperatur belassen. Man engt bei 40 °C im Vakuum zur Trockne ein und filtriert den sirupösen Rückstand mit Ethylacetat über Kieselgel. Nach Abdestillieren des Lösungsmittels kristallisiert das Produkt beim Verreiben mit Ether durch. Man erhält so 5,62 g (79 %) 1-Benzyl-(3R,4R)-3,4-dihydroxy-pyrrolidin2-on mit einem Schmelzpunkt von 77-78 °C und $[\alpha]_D^{20}$ + 15,6 ° (C = 1. $CH_3OH$).

Analog zu der im Beispiel 29 angegebenen Vorschrift erhält man auch die in Tabelle 4 aufgeführten Verbindungen aus den entsprechenden Vorstufen (Tabelle 3).

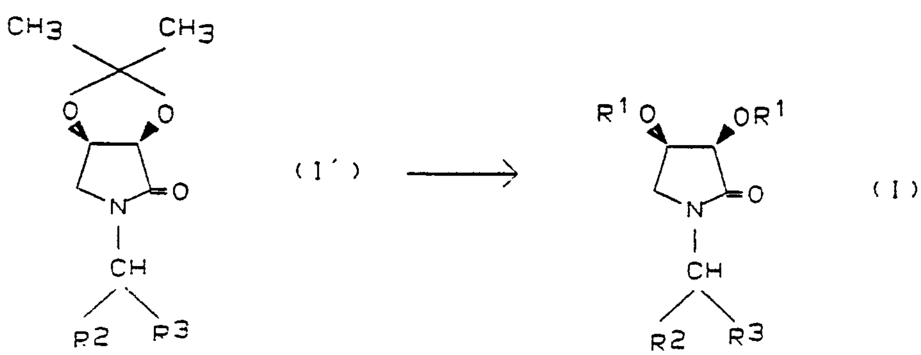

Tabelle 4

| Beispiel | Vorstufe Beispiel-Nr. | $R^1$ | $R^2$ | $R^3$ | Schmelzpunkt (°C) | Ausbeute (%) |
|---|---|---|---|---|---|---|
| 30 | 18 | H | H | 4-$NO_2$-$C_6H_4$-$CH_2$ | 159-160 | 98 |
| 31 | 19 | H | H | 4-Br-$C_6H_4$-$CH_2$ | 125-127 | 98 |
| 32 | 20 | H | H | 4-F-$C_6H_4$-$CH_2$ | 119-121 | 74 |
| 33 | 21 | H | H | 4-($CH_3$)-$C_6H_4$-$CH_2$ | 127-129 | 86 |
| 34 | 22 | H | H | 3-($OCH_3$)-$C_6H_4$-$CH_2$ | 106-108 | 67 |
| 35 | 23 | H | H | $CONH_2$ | 140-142 | 73 |

**Beispiel 36**

Herstellung von

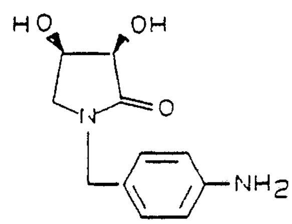

4,50 g (17,8 mmol) Produkt aus Beispiel 30 in 100 ml Methanol und 30 ml Wasser werden bei Normaldruck und Raumtemperatur in Gegenwart von 500 mg 10 proz. Palladiumkohle hydriert. Nach Aufnahme der berechneten Wasserstoffmenge wird das Produkt durch Filtration, Einengen zur Trockne und Kristallisation aus Ethylacetat/Ether gereinigt. Man erhält so 3,60 g (89 %) 1-(4-Aminobenzyl)-(3R,4R)-3,4-dihydroxypyrrolidin-2-on vom Schmelzpunkt 157-159 °C und $[\alpha]_D^{20}$ + 8,0° (C = 1, $CH_3OH$).

**Ansprüche**

1. 3,4-Dihydroxypyrrolidin-2-on-Derivate der Formel I

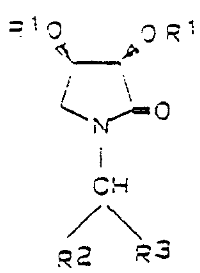

(I)

worin

$R^1$ Wasserstoff, $C_1$-$C_8$-Alkylcarbonyl oder worin die beiden $R^1$-Reste zusammen einen Isopropyliden-Rest darstellen

und

$R^2$ Wasserstoff, $C_1$-$C_7$-Alkyl, Aryl oder Heteroaryl bedeutet, wobei die Aryl- und Heteroarylreste

gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und

$R^3$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind oder $R^3$ einen Rest der Formel -CO-X-$R^4$ bedeutet, wobei

X Sauerstoff oder NH und

$R^4$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind und $R^4$ für den Fall, daß X NH bedeutet auch ein Rest der Formel --$(CH_2)_2$-$NR^5R^6$ sein kann, wobei $R^5$ und $R^6$ Wasserstoff oder $C_1$-$C_7$-Alkyl ist oder wobei $R^5$ und $R^6$ zusammen mit dem Stickstoffatom an dem sie gebunden sind einen Pyrrolidin-, Piperidin-, Piperazin- oder Morpholin-Ring bilden, wobei diese Pyrrolidin-, Piperazin-, Piperidin- und Morpholin-Reste ihrerseits gegebenenfalls mit $C_1$-$C_7$-Alkyl mono- oder disubstituiert sind

sowie deren physiologisch verträglichen Salze.

2. 3,4-Dihydroxypyrrolidin-2-on Derivate der Formel I gemäß Anspruch 1, dadurch gekennzeichnet, daß mindestens eine der nachfolgenden Bedingungen erfüllt ist:

$R^1$ bedeutet Wasserstoff

$R^2$ bedeutet Wasserstoff, Phenyl oder Thienyl, wobei die Phenyl- und Thienylreste gegebenenfalls mit $CF_3$, F, Cl, Br, J, $C_1$-$C_4$-Alkyl, $C_1$-$C_4$ Alkoxy, OH, COOH, COOMe, COOEt, $NO_2$ oder CN mono- oder disubstituiert sind

$R^3$ bedeutet Wasserstoff oder -CO-$NHR^4$ wobei $R^4$ Wasserstoff oder -$(CH_2)_2$-$NR^5R^6$ bedeutet, wobei $R^5$ und $R^6$ $C_1$-$C_3$-Alkyl ist oder wobei $R^5$ und $R^6$ zusammen mit dem Stickstoffatom an dem sie gebunden sind einen Di-$C_1$-$C_3$-Alkyl-substituierten Piperidin-Ring bilden.

4. 3,4-Dihydroxypyrrolidin-2-on-Derivate der Formel I gemäß Anspruch 2, dadurch gekennzeichnet, daß mindestens eine der nachfolgenden Bedingungen erfüllt ist

A) $R^1$ und $R^2$ bedeuten Wasserstoff

B) $R^1$ und $R^3$ bedeuten Wasserstoff.

4. Verfahren zur Herstellung von 3,4-Dihydroxypyrrolidin2-on-Derivaten der Formel I gemäß Anspruch 1, dadurch gekennzeichnet, daß man

a) ein Amin der Formel II

$R^2$ - CH - $R^3$ (II)
$NH_2$

worin $R^2$ und $R^3$ die in Anspruch 1 zu Formel I angegebenen Bedeutungen haben mit der Verbindung der Formel III

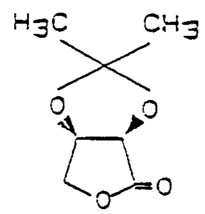

(III)

zu Amiden der Formel IV

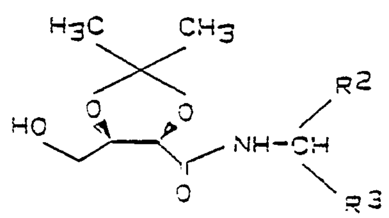

(IV)

worin $R^2$ und $R^3$ die in Anspruch 1 zu Formel I angegebenen Bedeutungen haben, umsetzt und anschließend die Amide der Formel IV in eine Verbindung der Formel V

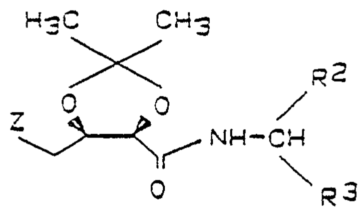

(V)

überführt, worin
$R^2$ und $R^3$ die in Anspruch 1 zu Formel I angegebenen Bedeutungen haben und Z eine nukleofuge Gruppe ist und dann die Verbindung der Formel V in die geschützten Pyrrolidinon-Derivate der Formel I′ überführt,

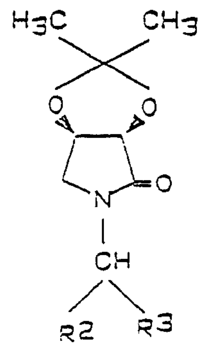

(I′)

worin $R^2$ und $R^3$ die in Anspruch 1 zu Formel I angegebenen Bedeutungen haben
oder daß man

b) (3R,4R)-3,4-Di-O-isopropylidenerythronsäurelactam (Formel VI)

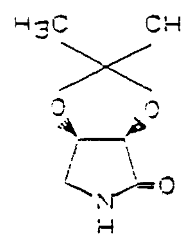

(VI)

mit einer Verbindung der Formel VII

$$R^2 - \overset{Z}{\overset{|}{CH}} - R^3 \quad (VII)$$

alkyliert, wobei $R^2$ und $R^3$ die in Anspruch 1 zu Formel I angegebenen Bedeutungen haben und Z eine nukleofuge Gruppe ist, wobei eine Verbindung der Formel I′ entsteht und daß man anschließend in den nach Verfahrensvarianten a) oder b) erhältlichen Verbindungen der Formel I′ gegebenenfalls die Hydroxy-Schutzgruppe in 3,4-Position des Pyrrolidinon-Rings abspaltet und die dabei entstandenen 3,4-Dihydroxy-pyrrolidin-2-one schließlich in die 3,4-Di-O-alkanoyl-pyrrolidin-2-one überführt und diese dann gegebenenfalls noch in ihre physiologisch verträglichen Salze überführt.

5. Arzneimittel, dadurch gekennzeichnet, daß es eine Verbindung der Formel I gemäß Anspruch 1 oder ein physiologisch verträgliches Salz dieser Verbindung in einer therapeutisch wirksamen Menge, gegebenenfalls zusammen mit üblichen pharmazeutischen Hilfs- oder Trägerstoffen enthält.

6. Arzneimittel nach Anspruch 5, dadurch gekennzeichnet, daß es nootrop wirksame Mengen einer Verbindung nach Anspruch 1 enthält.

7. Verwendung einer Verbindung der Formel I gemäß Anspruch 1 oder eines physiologsich verträglichen Salzes dieser Verbindung zur Herstellung eines Arzneimittels.

8. Verwendung einer Verbindung der Formel I gemäß Anspruch 1 oder eines physiologisch verträglichen Salzes dieser Verbindung als Nootropikum.

9. Verbindungen der Formel I gemäß Anspruch 1 zur Verwendung als Nootropikum.

10. Verbindungen der Formel IV gemäß Anspruch 4, wobei $R^2$ und $R^3$ die in Anspruch 1 zu Formel I angegebenen Bedeutungen haben.

11. Verbindungen der Formel V gemäß Anspruch 4, wobei $R^2$ und $R^3$ die in Anspruch 1 zu Formel I angegebenen Bedeutungen haben und Z die in Anspruch 4 zu Formel V angegebene Bedeutung hat.

Patentanspruch für folgenden Vertragsstaat ES, GR

1. Verfahren zur Herstellung von 3,4-Dihydroxypyrrolidin-2-on-Derivatender Formel I

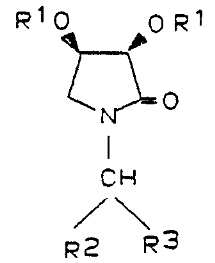

( I )

worin

$R^1$ Wasserstoff, $C_1$-$C_8$-Alkylcarbonyl oder worin die beiden $R^1$-Reste zusammen einen Isopropyliden-Rest darstellen

und

$R^2$ Wasserstoff, $C_1$-$C_7$-Alkyl, Aryl oder Heteroaryl bedeutet, wobei die Aryl- und Heteroarylreste gegebenenfalls mit $CF_3$, F, Cl, Br, J, Cl-$C_4$-Alkyl, $C_1$-$C_4$-Alkoxy, OH, COOH, COOMe, COOEt, $NO_2$ oder CN mono- oder disubstituiert sind und

$R^3$ Wasserstoff $C_1$-$C_7$-Alkyl, Aryl oder Heteroaryl bedeutet, wobei die Aryl- und Heteroarylreste gegebenenfalls mit $CF_3$, F Cl, Br, J, C -$C_4$-Alkyl, $C_1$-$C_4$-Alkoxy OH, COOH, COOMe, COOEt $NO_2$ oder CN mono- oder disubstituiert sind oder $R^3$ einen Reste der Formel -CO-X-$R^4$ bedeutet wobei

X Sauerstoff oder NH und

$R^4$ Wasserstoff, $C_1$-$C_7$-Alkyl, Aryl oder Heteroaryl bedeutet, wobei die Aryl- und Heteroarylreste gegebenenfalls mit $CF_3$ F, Cl, Br, J, $C_1$-$C_4$-Alkyl, $C_1$-$C_4$ Alkoxy, OH, COOH, COOMe COOEt-, $NO_2$ oder CN mono- oder disubstituiert sind und $R^4$ für den Fall, daß X NH bedeutet auch ein Rest der Formel $-(CH_2)_2-NR^5R^6$ sein kann, wobei $R^5$ und $R^6$ Wasserstoff oder $C_1$-$C_7$-Alkyl ist oder wobei $R^5$ und $R^6$ zusammen mit dem Stickstoffatom an dem sie gebunden sind einen Pyrrolidin-, Piperidin-, Piperazin- oder Morpholin-Ring bilden. wobei diese Pyrrolidin- Piperazin- Piperidin- und Morpholin-Reste ihrerseits gegebenenfalls mit $C_1$-$C_7$-Alkyl mono- oder disubstituiert sind

sowie deren physiologisch verträglichen Salzen dadurch gekennzeichnet, daß man

a) in Amin der Formel II

$R^2$ - CH - $R^3$ (II)

$NH_2$

worin $R^2$ und $R^3$ die zu Formel I angegebenen Bedeutungen haben mit der Verbindung der Formel III

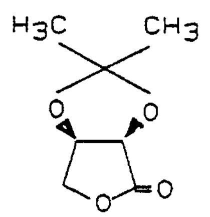

( I I I )

zu Amiden der Formel IV

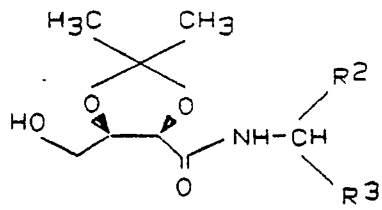

(IV)

worin $R^2$ und $R^3$ die zu Formel I angegebenen Bedeutungen haben, umsetzt und anschließend die Amide der Formel IV in eine Verbindung der Formel V

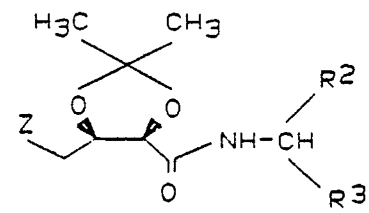

(V)

überführt, worin
$R^2$ und $R^3$ die zu Formel I angegebenen Bedeutungen haben und Z eine nukleofuge Gruppe ist und dann die Verbindung der Formel V in die geschützten Pyrrolidinon-Derivate der Formel I′ überführt

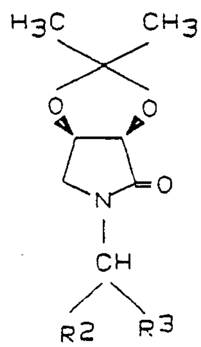

(I′)

worin $R^2$ und $R^3$ die zu Formel I angegebenen Bedeutungen haben
oder daß man
b) (3R,4R)-3,4-Di-O-isopropylidenerythronsäurelactam (Formel VI)

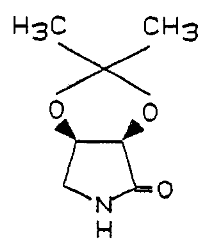

(VI)

mit einer Verbindung der Formel VII

Z
$R^2$ - CH - $R^3$ (VII)

alkyliert, wobei $R^2$ und $R^3$ di zu Formel I angegebenen Bedeutungen haben und Z eine nukleofugen Gruppe ist, wobei eine Verbindung der Formel I′ entsteht und daß man anschließend in den nach Verfahrensvarianten a) oder b) erhältlichen Verbindungen der Formel I′ gegebenenfalls die HydroxySchutzgruppe in 3,4-Position des Pyrroldinon-Rings abspaltet und die dabei entstandenen 3,4-Dihydroxy pyrrolidin-2-one schließlich in die 3,4-Di-O-alkanoyl-pyrrolidin-2-one überführt und diese dann gegebenenfalls noch in ihre physiologisch verträglichen Salze uberführt.

2. Verfahren zur Herstellung von 3,4-Dihydroxypyrrolidin2-on-Derivatender Formel I gemäß Anspruch 1, dadurch gekennzeichnet, daß mindestens eine der nachfolgenden Bedingungen erfüllt ist:
$R^1$ bedeutet Wasserstoff $R^2$ bedeutet Wasserstoff, Phenyl oder Thienyl, wobei die Phenyl- und Thienylreste gegebenenfalls mit $CF_3$, F, Cl, Br, J, $C_1$-$C_4$-Alkyl, $C_1$-$C_4$-Alkoxy, OH, COOH, COOMe, COOEt, $NO_2$ oder CN mono- oder disubstituiert sind
$R^3$ bedeutet Wasserstoff oder $-CO-NHR^4$ wobei $R^4$ Wasserstoff oder $-(CH_2)_2-NR^5R^6$ bedeutet, wobei $R^5$ und $R^6$ $C_1$-$C_3$-Alkyl ist oder wobei $R^5$ und $R^6$ zusammen mit dem Stickstoffatom an dem sie gebunden sind einen Di-$C_1$-$C_3$-Alkyl-substituierten Piperidin-Ring bilden.

3. Verfahren zur Herstellung von 3,4-Dihydroxypyrrolidin-2-on-Derivaten der Formel I gemäß Anspruch 1. dadurch gekennzeichnet, daß mindestens eine der nachfolgenden Bedingungen erfüllt ist:
A) $R^1$ und $R^2$ bedeuten Wasserstoff
B) $R^1$ und $R^3$ bedeuten Wasserstoff.

4. Verbindungen der Formel I sofern sie gemäß einem oder mehreren der Ansprüche 1-3 hergestellt worden sind.

5. Verfahren zur Herstellung eines Arzneimittels, dadurch gekennzeichnet daß man eine Verbindung der Formel I gemäß Anspruch 1 oder ein physiologisch verträgliches Salz dieser Verbindung in einer therapeutisch wirksamen Menge, gegebenenfalls zusammen mit üblichen pharmazeutischen Hilfs- oder Trägerstoffen in eine geeignete Darreichungsform bringt.

6.Verfahren zur Herstellung von Arzneimitteln nach Anspruch 5, dadurch gekennzeichnet, daß es nootrop wirksame Mengen einer Verbindung nach Anspruch 1 enthält.

7. Verwendung einer Verbindung der Formel I gemäß Anspruch 1 oder eines physiologisch verträglichen Salzes dieser Verbindung zur Herstellung eines Arzneimittels.

8. Verwendung einer Verbindung der Formel I gemaß Anspruch oder eines physiologisch vertraglichen Salzes dieser Verbindung als Nootropikum.

9. Verfahren gemaß Anspruch I zur Herstellung von Verbindungen der Formel IV gemäß Anspruch 1.

10. Verfahren gemaß Anspruch 1 zur Herstellung von Verbindungen der Formel V gemäß Anspruch 1.

Europäisches Patentamt

# EUROPÄISCHER RECHERCHENBERICHT

Nummer der Anmeldung

EP 89 10 6625

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | EP-A-0 071 216 (F. HOFFMANN-LA ROCHE & CO.)<br>* Ansprüche 1,12,13,16,17 * | 1,5-9 | C 07 D 207/273<br>C 07 D 491/04<br>C 07 D 409/06<br>C 07 D 317/32<br>A 61 K 31/40 |
| A | DE-A-2 635 853 (I.S.F. S.P.A.)<br>* Ansprüche 1,13; Seite 6, Zeilen 23-30 * | 1,5-9 | |
| A | DE-A-2 758 939 (I.S.F. S.P.A.)<br>* Ansprüche 1,9; Seite 10, Zeilen 21-27 * | 1,5-9 | |
| A | DE-A-2 923 975 (A. NATTERMANN & CIE GMBH)<br>* Ansprüche 1,3; Seite 7, Zeile 21 - Seite 8, Zeile 20 * | 1,5-9 | |
| A | GB-A-2 040 933 (A. H. ROBINS COMPANY)<br>* Seite 1, Zeile 1 - Seite 2, Zeile 34 * | 1,5-7 | |

RECHERCHIERTE SACHGEBIETE (Int. Cl.4)

C 07 D 207/00
C 07 D 491/00
C 07 D 409/00
C 07 D 317/00
A 61 K 31/00

Der vorliegende Recherchenbericht wurde für alle Patentansprüche erstellt

| Recherchenort | Abschlußdatum der Recherche | Prüfer |
|---|---|---|
| BERLIN | 17-07-1989 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)